# FASCICULE DE BREVET EUROPEEN

(11) **EP 0 172 117 B1**
(45) Date de publication et mention de la délivrance du brevet: **03.02.1993**
(21) Numéro de dépôt: 85420129.0
(22) Date de dépôt: 12.07.1985
(51) Int. Cl.: A61M 1/10, G01L 9/00

(54) **Pompe péristaltique équipée d'un dispositif de mesure de pression**
Peristaltische Pumpe mit einer Druckmessungsvorrichtung
Peristaltic pump provided with pressure measuring means

(30) Priorité: 07.08.1984 IT 5370984 U
(43) Date de publication de la demande: 19.02.1986
(73) Titulaire: HOSPAL AG, CH-4055 Basel (CH)
(72) Inventeur: Aldrovandi, Mauro, I-41037 Mirandola (Modena) (IT); Cianciavicchia, Domenico, Teramo (IT); Pedrazzi, Renato, Mirandola (IT)
(74) Mandataire: Kerneis, Daniéle

(56) Documents cités:
- WO-A-80/02376
- DE-A- 2 415 528
- DE-A- 2 823 670
- FR-A- 2 198 759
- US-A- 3 841 157
- US-A- 3 949 734
- US-A- 4 187 720

## Description

La présente invention concerne un appareil pour la circulation du sang le long d'un conduit tubulaire, en particulier, du sang qui s'écoule en circulation extracorporelle, par exemple pour être purifié par une opération de dialyse.

L'appareil auquel se rapporte la présente invention est du type comprenant essentiellement une pompe péristaltique munie d'un rotor qui agit au moyen de rouleaux sur la surface extérieure d'un tube déformable élastiquement disposé en série le long d'une ligne tubulaire extracorporelle pour le sang et comprenant en outre un dispositif capable de fournir une indication sur la pression du sang qui s'écoule à l'amont de la pompe péristaltique elle-même.

En général, ce genre d'appareil comprend un réservoir tubulaire, souvent appelé amortisseur de pulsations et construit en matériau déformable élastiquement qui est relié en série à la ligne extracorporelle de sang ainsi que cela est décrit dans le brevet US 3 949 734. Ce réservoir peut, en fonctionnement, être assemblé à l'intérieur d'un corps constitué par une cuvette fermée à la partie supérieure par un couvercle. L'appareil peut comprendre en outre un micro-interrupteur monté sur la paroi du fond de la cuvette afin de relever les déformations induites par la pression du sang, et une vis portée par le couvercle manoeuvrable par l'opérateur pour déformer partiellement le réservoir et pouvoir ainsi déterminer à l'avance un seuil d'intervention pour le micro-interrupteur.

On a remarqué que les appareils du type décrit ci-dessus présentent de nombreux inconvénients découlant de l'emploi de ce réservoir. En fait, celui-ci contient, en fonctionnement, une masse de sang représentant un pourcentage significatif de la capacité de la ligne extracorporelle et ceci entraîne une inertie élevée, le temps de réponse entre les variations de pression et la signalisation étant souvent trop long. Les parois du réservoir pour des raisons pratiques de fabrication, ne peuvent pas être exécutées en matériau présentant un degré élevé d'élasticité, ce qui influe négativement sur la sensibilité du dispositif. Le système de régulation du seuil d'intervention au moyen de la vis précitée nécessite une adaptation de la structure du réservoir influençant la valeur de la sensibilité et exigeant l'intervention de l'opérateur pour réajuster le seuil d'intervention chaque fois que l'on utilise une nouvelle ligne, puisque on ne peut enlever le réservoir qu'après avoir dévissé la vis portée par le couvercle. L'adoption du réservoir entraîne de très fortes variations de la section transversale du flux de sang s'écoulant dans le conduit extracorporel, puisque la section droite de la ligne est considérablement inférieure à celle du réservoir.

De telles variations de section droite, outre qu'elles rendent possible l'aspiration d'air au niveau de la jonction du réservoir et de la ligne, provoquent aussi un mouvement turbulent du sang pouvant l'endommager en provoquant par exemple une hémolyse des globules rouges du sang. Enfin il est opportun de noter que le réservoir est toujours un composant à produire et à assembler, par collage, en série à la ligne extracorporelle de sang et que ceci entraîne des coûts de production et de main-d'oeuvre non négligeables.

Un but de la présente invention est de proposer un appareil pour la circulation d'un liquide, par exemple de sang, le long d'une ligne tubulaire, qui évite les inconvénients présentés par les appareils de types connus et énoncés ci-avant.

Ce but est atteint par la présente invention qui concerne un appareil pour la circulation extra corporelle du sang comprenant un conduit tubulaire, une pompe ainsi que des moyens pour mesurer la pression du sang à l'intérieur de la ligne, les moyens comprenant :
- un élément de référence et un élément mobile entre lesquels il est possible d'interposer une partie du conduit, ladite partie étant en amont de la pompe,
- des moyens de mesure qui relèvent la position relative prise par ledit élément mobile par rapport audit élément de référence et qui émettent un signal électrique dépendant de la déformation dudit tronçon causée, en fonctionnement, par la pression dudit sang, caractérisé en ce que la pompe est une pompe péristaltique possédant un rotor qui agit au moyen de rouleaux sur la surface extérieure d'un tube déformable élastiquement placé en série avec ledit conduit tubulaire et en ce que la partie du conduit sur la quelle les moyens pour mesurer la pression sont montés est constituée par un tronçon du tube non soumis à l'action des rouleaux en amont de la pompe.

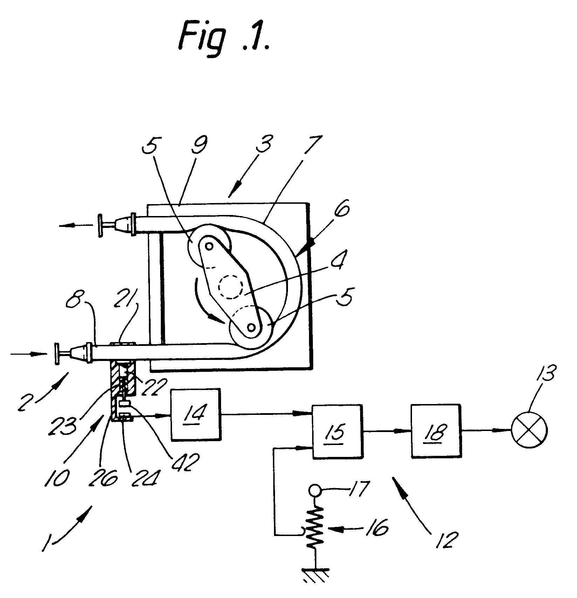

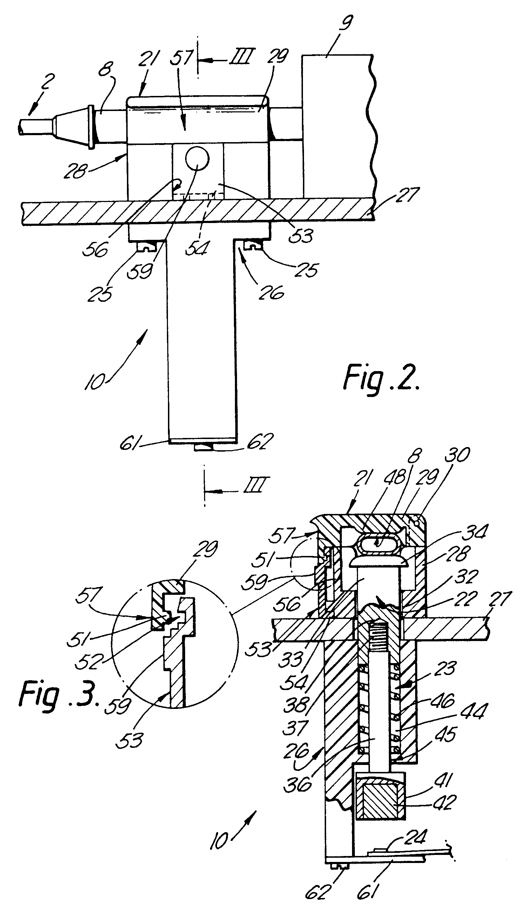

Pour une meilleure compréhension de la présente invention, on va maintenant décrire une forme préférée de réalisation, à simple titre d'exemple non limitatif et en référence aux dessins ci-joints, dans lesquels :
. la figure 1 est une vue schématique et partiellement en coupe d'un appareil construit selon la présente invention ;
. la figure 2 est une vue en élévation et à échelle agrandie d'une partie de la figure 1 ; et
. la figure 3 est une vue en coupe selon le plan III - III de la figure 2.

En se référant particulièrement à la figure 1, on désigne par la référence 1, l'ensemble de l'appareil pour la circulation du sang le long d'une ligne tubulaire 2. L'appareil (1) comprend essentiellement une pompe péristaltique (3) dotée d'un rotor (4) qui agit, au moyen des rouleaux (5) sur la surface extérieure d'un premier tronçon (7) d'un tube (6) constitué par un matériau déformable élastiquement tel par exemple qu'un chlorure de polyvinyle plastifié (PVC) et placé en série le long de la ligne tubulaire (2) citée ci-avant. L'appareil (1) comprend en outre un dispositif (10) qui est disposé extérieurement à un second tronçon (8) du tube (6) qui s'étend à l'extérieur du corps en forme de cuvette (9) de la pompe péristaltique (3).

Comme il est décrit plus en détail par la suite en se référant aux figures 2 et 3, le dispositif (10) est capable d'émettre un signal électrique dépendant de la déformation induite, en fonctionnement, dans le tronçon (8) du tube (6) par effet de la pression du liquide qui s'écoule à l'intérieur de la partie de la ligne tubulaire (2) disposée à l'amont de la pompe péristaltique (3).

L'appareil (1) comprend enfin un circuit électrique indiqué dans son ensemble par la référence (12), lequel compare le signal électrique émis par le dispositif (10) avec un signal de référence règlable dans le but de fournir un signal d'alarme à une lampe (13) quand l'écartement entre ces deux signaux sort des limites de sécurité prédéterminées. Plus particulièrement le circuit (12) comprend un amplificateur (14) qui recoit le signal électrique émis par le dispositif (10) et qui envoie un tel signal convenablement amplifié sur la première entrée d'un circuit comparateur (15) à la seconde entrée qui reçoit le signal règlable précité fourni par le curseur d'un potentiomètre (16) dont les extrémités opposées sont reliées respectivement à la masse et à une source d'alimentation de type continu reliée à une borne (17). La sortie du circuit comparateur (15) est enfin reliée, au moyen d'un amplificateur (18) supplémentaire, à la lampe de signalisation (13).

On décrit maintenant la structure du dispositif (10) en se référant plus particulièrement aux figures (1) et (3). Un tel dispositif comprend essentiellement un élément de référence (21) et un élément mobile (22) entre lesquels on peut interposer le tronçon (8) du tube précité (6); des moyens élastiques (23) qui transmettent à l'élément mobile (22) une poussée tournée vers l'élément de référence (21) en direction radiale par rapport au tronçon (8) du tube (6); et des moyens de mesure (24) qui relèvent la position relative de l'élément mobile (22) par rapport à l'élément de référence (21) et émettent en conséquence le signal électrique utilisé dans le circuit (12).

Plus particulièrement, le dispositif (10) présente essentiellement un support tubulaire (26) relié à un élément (28) en forme de U au moyen de vis (25) qui traversent par des trous non représentés,une plaque (27) qui supporte aussi le corps (9) de la pompe péristaltique (3). L'élément (28) en forme de U, formant une cuvette est fermé à la partie supérieure par un couvercle (29) qui constitue essentiellement ledit élément de référence (21) et qui peut pivoter autour d'un axe (30). L'élément (28) en forme de cuvette présente à sa base un trou (32) dans lequel coulisse axialement l'élément mobile précité (22).

Ce dernier est constitué essentiellement par un piston présentant une partie supérieure cylindrique (33) ayant un diamètre légèrement inférieur au diamètre du trou (32) cité ci-dessus et présentant une tête (34) destinée à coopérer, en fonctionnement avec la surface externe du tronçon (8) du tube (6) déformable et élastique. La surface de la tête (34) en face du tronçon de tube (8) est plane avec à la périphérie une surface de raccordement essentiellement sphérique. L'élément mobile (22) présente en outre une partie inférieure cylindrique (36), coaxiale à la partie (33), mais présentant un diamètre inférieur. La partie inférieure (36) possède une extrémité filetée (37) engagée dans un trou taraudé correspondant (38) ménagé dans la partie supérieure (33). A l'opposé de l'extrémité (37), la partie inférieure cylindrique (36) porte une tête (41) formant cuvette à l'intérieur de laquelle est logé un aimant permanent (42) capable d'agir sur lesdits moyens de mesure (24), comme décrit par la suite.

Le corps tubulaire (26) du dispositif (10) présente en outre, une chambre cylindrique (44) percée dans sa partie inférieure d'un trou (45) dans lequel coulisse la partie inférieure cylindrique (36) de l'élément mobile (22). Le diamètre intérieur de la chambre (44) est légèrement supérieur au diamètre de la partie cylindrique (33); la chambre (44) exerce donc la fonction de guide pour les déplacements axiaux de cet élément cylindrique (33) et elle reçoit en particulier un ressort à boudin (46) coaxial à l'élément cylindrique (36) de diamètre inférieur, afin de transmettre à la partie supérieure cylindrique (33) la poussée précitée tournée vers l'élément de référence (21) et en direction radiale par rapport au troncon (8) du tube (6). Ce tronçon (8) est donc soumis à une action élastique due à la poussée exercée par le ressort (46) qui, en fonctionnement, le déforme comme indiqué figure 3, appuyant d'un côté sur la surface plane et circulaire de la tête (34) de la partie supérieure cylindrique (33) de l'élément mobile (22) et, du côté opposé sur une surface plane circulaire correspondante offrant un relief (48) s'étendant depuis la partie tournée vers l'intérieur du corps du couvercle (29).

La liaison entre le couvercle (29) et la partie (28) correspondante en forme de cuvette, s'effectue, en plus de l'axe (30), par encliquetage d'une dent (51) du couvercle (29) avec une cavité correspondante (52) creusée à l'extrémité d'un élément (53) déformable élastiquement et relié à la base avec l'élément en forme de cuvette (28). Plus particulièrement, la liaison entre l'élément (53) et la partie formant cuvette (28) se produit essentiellement en forçant la base (54) en forme de queue d'aronde de l'élément (53) dans une cavité ménagée à la périphérie de la paroi du fond de la cuvette de l'élément (28). En correspondance avec l'élément déformable élastiquement (53), la paroi latérale du corps formant cuvette (28) présente un siège essentiellement rectangulaire désigné par la référence (56), afin de recevoir pratiquement tout l'élément (53), faisant en sorte que ce dernier se trouve essentiellement sur le même plan que la surface latérale correspondante (57) du couvercle (29).

On observe enfin que à proximité de la cavité (52) et de la partie lui faisant face vers l'extérieur, l'élément (53) présente un relief cylindrique (59) dont le but est d'indiquer à l'utilisateur la région la plus opportune pour exercer une pression sur l'élément (53) lui-même pour obtenir le désaccouplement entre la dent (51) et la cavité (52) et ainsi l'ouverture du couvercle (29).

On précise aussi que les moyens de mesure (24) sont supportés par un étrier (61) relié au corps (26) du dispositif (10) au moyen d'une vis (62). De tels moyens de mesure sont donc disposés en face de et sur le même axe que l'aimant permanent (42) et ils sont en mesure d'émettre un signal électrique dépendant de l'intensité du champ magnétique dans lequel ils sont placés, signal électrique qui dépend évidemment de la distance entre l'aiment (42) et les moyens de mesure (24). De préférence, ces moyens de mesure comportent un transducteur à effet "Hall".

Le fonctionnement de l'appareil (1) s'effectue de la manière suivante. Dans la phase de mise en place de la ligne extracorporelle de sang, on prend le tronçon (8) du tube (6) déformable élastiquement, situé en deçà de la pompe péristaltique (3), dans la partie qui sera soumise à une aspiration sous l'effet de la rotation du rotor (4). Puis on introduit simplement le tronçon (8) à l'intérieur de la pièce formant la cuvette (28) et l'on ferme le couvercle (29) en exerçant une pression axiale dans la partie opposée à celle qui tourne autour de l'axe (30). De cette manière, la dent (51) fait fléchir élastiquement vers l'intérieur l'élément (53) déformable jusqu'à ce qu'il occupe la cavité correspondante. Dans de telles conditions, le tronçon (8) se trouve disposé entre le couvercle (29) et la tête plate (34) de l'élément mobile (22) et est en outre sujet à la poussée radiale qu'il reçoit du ressort (46).

Dans des conditions normales la pompe péristaltique (3) déplace le flux du sang le long de la ligne (2), en l'aspirant depuis la partie où est situé le tronçon (8) et en le refoulant dans la partie opposée du tube (6). Dans le cas où l'on observe à l'amont de la pompe péristaltique (3) une obstruction de quelque importance, le long de la ligne (2), on observe aussi une diminution de la pression du sang contenu à l'intérieur de la ligne (2) elle-même et en particulier aussi à l'intérieur du tronçon (8). Puisque celui-ci est réalisé dans un matériau particulièrement déformable du point de vue élastique, il se déforme radialement et l'action du ressort (46) a pour effet de maintenir la surface de la tête (34) au contact avec la surface correspondante du tronçon (8), ce qui a pour conséquence d'augmenter la distance entre l'aimant (42) et les moyens de mesure (24). Comme on l'a déjà dit, le signal électrique émis par ceux-ci dépend de la valeur du champ magnétique dans lequel ils baignent, il subit, dans ce cas spécifique, une réduction d'amplitude. Un tel signal électrique, après avoir été amplifié par l'amplificateur (14), est comparé par le comparateur (15) avec le signal de référence transmis par le curseur du potentiomètre (16) et, dans le cas où l'écart entre les deux signaux sort de limites prédéterminées, le circuit comparateur (15) émet un signal d'alarme qui provoque l'éclairage de la lampe (13) au moyen de l'amplificateur (18). Le règlage du seuil d'intervention dans le circuit comparateur (15) peut s'effectuer simplement en agissant sur le curseur du potentiomètre (16) et en règlant celui-ci jusqu'à obtenir la valeur de seuil désirée.

De l'examen des caractéristiques de l'appareil (1) décrit ci-dessus, les avantages qu'il permet d'obtenir apparaissent de manière évidente. En fait la quantité de sang utilisée pour le contrôle de la pression est maintenant très nettement inférieure à celle contenue dans les réservoirs utilisés antérieurement, puisqu'elle est limitée à celle contenue dans le tronçon (8) du tube déformable (6). Il en résulte une moindre inertie qui permet d'avoir une réponse rapide avec signalisation immédiate des déformations du tronçon (8) induites par les variations de pression hors des limites admissibles.

On obtient aussi une rapide reprise des conditions normales de fonctionnement grâce, soit à l'élasticité importante des parois de tronçon (8), soit à la moindre quantité de sang circulant entre la zône d'entrée de la pompe (6) et la zône d'accès vasculaire de la ligne extracorporelle de sang (2). La sensibilité du dispositif (10) est sensiblement améliorée par suite de la grande élasticité des parois du tronçon (8) et elle n'est pas influencée par les règlages des seuils qui sont effectués électroniquement.

En outre les opérations de mise en place et d'enlèvement de la ligne extracorporelle de sang (2) sont encore simplifiées, car il n'est plus nécessaire de répéter chaque fois l'étalonnage en vissant et dévissant la vis disposée dans le couvercle des appareils connus. Il en résulte qu'il n'y a plus de changements de section droite en dehors de ceux exigés par le raccordement entre la ligne (2) et le tube (6). Les problèmes connus précités dus à un collage imparfait des jonctions entre le réservoir et la ligne dans la pompe (3) ne subsistent plus, de même que les inconvénients (hémolyse, etc...) dus à l'établissement d'un écoulement turbulent du sang. Enfin il est évident que, la ligne n'exigeant plus l'existence d'un réservoir, elle est évidemment simplifiée et n'entraîne que des coùts de production et de main-d'oeuvre réduits.

Selon une variante les moyens de mesure (24) pourraient être construits convenablement à l'aide d'un interrupteur magnétique, du type connu sous le nom de "Reed" , auquel cas ils pourraient être montés sur un étrier dont la position par rapport à l'aiment (42) serait règlable axialement d'une manière fine et précise. Le transducteur (24) et l'aimant (42) pourraient aussi être remplacés par un transformateur différentiel ou par un transducteur de type potentiométrique, ou encore par un transducteur de type optico-électronique, lequel comporterait par exemple un dispositif optique à réflexion, etc... afin d'obtenir des formes de réalisation équivalentes à celle décrite ci-dessus.

Une autre forme de réalisation pourrait prévoir d'installer un instrument de signalisation de type analogique immédiatement à la sortie du circuit amplificateur (14), en remplacement du comparateur (15), du potentiomètre (16), de l'amplificateur (18) et de la lampe de signalisation (13). Mais dans un tel cas on contrôlerait périodiquement les indications fournies par un tel instrument.

## Revendications

1. Appareil (1) pour la circulation extra corporelle du sang comprenant un conduit tubulaire (2), une pompe (3) ainsi que des moyens pour mesurer la pression du sang à l'intérieur du conduit (2), les moyens comprenant :
- un élément de référence (21) et un élément mobile (22) entre lesquels il est possible d'interposer une partie du conduit (2), ladite partie étant en amont de la pompe (3),
- des moyens de mesure (24) qui relèvent la position relative prise par ledit élément mobile (22) par rapport audit élément de référence (21) et qui émettent un signal électrique dépendant de la déformation dudit tronçon (8) causée, en fonctionnement, par la pression dudit sang,
caractérisé en ce que la pompe est une pompe (3) péristaltique possédant un rotor (4) qui agit au moyen de rouleaux (5) sur la surface extérieure d'un tube (6) déformable élastiquement placé en série avec ledit conduit tubulaire (2) et en ce la partie du conduit (2) sur laquelle les moyens pour mesurer la pression sont montés est constituée par un tronçon (8) du tube (6) non soumis à l'action des rouleaux (5) en amont de la pompe (3).

2. Appareil selon la revendication 1, caractérisé par le fait que ledit dispositif (10) comprend des moyens de comparaison (15) dudit signal électrique avec un signal électrique de référence et d'émission d'un signal d'alarme en correspondance avec le relèvement d'un écart prédéterminé dudit signal électrique par rapport audit signal de référence.

3. Appareil selon la revendication (2), caractérisé par le fait que lesdits moyens de comparaison et d'émission d'un signal d'alarme comprennent un circuit comparateur à seuil (15).

4. Appareil selon l'une des revendications 2 ou 3, caractérisé par le fait qu'il comprend des moyens (16) de règlage dudit signal de référence.

5. Appareil selon la revendication 4, caractérisé par le fait que lesdits moyens de règlage comprennent un potentiomètre (16) et un curseur relié à l'entrée dudit circuit comparateur (15).

6. Appareil selon l'une quelconque des revendications précédentes, caractérisé par le fait que ledit élément de référence (21) est constitué essentiellement par le couvercle (29) d'un corps formant cuvette (28) à l'intérieur de laquelle on reçoit ledit tronçon (8) du tube (6); ledit élément mobile (22) étant essentiellement constitué par un piston (33) coulissant axialement à l'intérieur d'un trou (32) ménagé dans une paroi à la base dudit corps formant cuvette et dans une position faisant face audit couvercle (29).

7. Appareil selon la revendication 6, caractérisé par le fait que ledit piston (33) présente une tête (34) avec une surface centrale circulaire plane capable de coopérer avec une surface correspondante externe dudit tronçon (8) du tube (6).

8. Appareil selon la revendication 7, caractérisé par le fait que ledit couvercle (29) présente dans la partie tournée vers l'intérieur, un relief (48) essentiellement cylindrique, capable de constituer une surface circulaire plane semblable à celle présentée par la tête élargie (34) dudit piston (33).

9. Appareil selon une quelconque des revendications 6 à 8, caractérisé par le fait que l'accouplement entre ledit couvercle (29) et ledit élément en forme de cuvette (28) est à enclenchement.

10. Appareil selon la revendication 9, caractérisé par le fait que ledit accouplement à enclenchement est obtenu par l'emploi d'une dent (51) dont est muni le couvercle (29) avec une cavité (52) correspondante ménagée dans un élément (53) déformable élastiquement relié audit élément en forme de cuvette (28) dudit dispositif (10).

11. Appareil selon une des revendications 6 à 10, caractérisé en ce que ledit dispositif (10) capable de fournir les valeurs de la pression du liquide comprend des moyens élastiques (23) qui transmettent audit élément mobile (22) une poussée s'exerçant vers ledit élément de référence (21) en direction radiale par rapport audit tronçon (8) du tube (6) et en direction axiale par rapport audit piston (33).

12. Appareil selon la revendication 11, caractérisé par le fait qu'audit piston (33) est associé un élément cylindrique inférieur (36) dont la position axiale relative est relevée par des moyens de mesure (24).

13. Appareil selon la revendication 12, caractérisé par le fait que lesdits moyens élastiques (23) comprennent un ressort à boudin (46) coaxial audit élément cylindrique inférieur (36).

14. Appareil selon la revendication 12 ou 13, caractérisé par le fait que ledit élément cylindrique inférieur (36) est muni à une extrémité d'un aimant permanent et que lesdits moyens de mesure (24) comprennent au moins un transducteur de type électromagnétique.

15. Appareil selon la revendication 14, caractérisé par le fait que ledit transducteur électromagnétique est un transducteur à effet "Hall".

16. Appareil selon la revendication 14, caractérisé par le fait que ledit transducteur électromagnétique est un interrupteur avec contacts à action magnétique.

17. Appareil selon l'une quelconque des revendications 1 à 13, caractérisé par le fait que lesdits moyens de mesure (24) comprennent un transformateur différentiel dont l'accouplement dépend de la position dudit élément mobile (22).

18. Appareil selon l'une quelconque des revendications 1 à 13, caractérisé par le fait que lesdits moyens de mesure (24) comprennent un transducteur de type potentiométrique.

19. Appareil selon l'une quelconque des revendications 1 à 13, caractérisé par le fait que lesdits moyens de mesure (24) comprennent au moins un transducteur de type optico-électronique.

## Claims

1. Apparatus (1) for the extracorporeal circulation of the blood comprising a tubular conduit (2), a pump (3) as well as means for measuring the blood pressure inside the conduit (2), these means comprising :
- a reference element (21) and a movable element (22) between which it is possible to interpose a portion of the conduit (2), the said portion being upstream from the pump (3),
- measurement means (24) which note the relative position taken up by the said movable element (22) relative to the said reference element (21) and which emit an electric signal depending on the deformation of the said section (8) caused in operation by the pressure of the said blood,
characterized in that the pump is a peristaltic pump (3) having a rotor (4) which acts by means of rollers (5) on the external surface of an elastically deformable tube (6) placed in series with the said tubular conduit (2), and in that the portion of the conduit (2) whereon the means for measuring the pressure are mounted is constituted by a section (8) of the tube (6) not subjected to the action of the rollers (5) upstream from the pump (3).

2. Apparatus according to claim 1, characterized in that the said device (10) includes means (15) for comparing the said electric signal with an electric reference signal and for emitting an alarm signal in accordance with the finding of a predetermined divergence of the said electric signal as compared with the said reference signal.

3. Apparatus according to claim 2, characterized in that the said comparison means and alarm signal emission means include a threshold comparator circuit (15).

4. Apparatus according to one of claims 2 or 3, characterized in that it includes means (16) for adjusting the said reference signal.

5. Apparatus according to claim 4, characterized in that the said adjustment means include a potentiometer (16) and a slider connected to the input of the said comparator circuit (15).

6. Apparatus according to any one of the preceding claims, characterized in that the said reference element (21) is constituted in essence by the lid (29) of a body forming a pan (28) within which the said section (8) of the tube (6) is held; the said movable element (22) being constituted in essence by a piston (33) axially sliding within a hole (32) arranged in a wall at the base of the said body forming a pan and in a position facing the said lid (29).

7. Apparatus according to claim 6, characterized in that the said piston (33) has a head (34) with a flat circular central surface capable of cooperating with a corresponding external surface of the said section (8) of the tube (6).

8. Apparatus according to claim 7, characterized in that the said lid (29) has, in the part turned towards the inside, a substantially cylindrical relief (48) capable of constituting a flat circular surface similar to that presented by the enlarged head (34) of the said piston (33).

9. Apparatus according to any one of claims 6 to 8, characterized in that the connection between the said lid (29) and the said pan-shaped element (28) is a snap coupling.

10. Apparatus according to claim 9, characterized in that the said snap coupling is obtained by the use of a tooth (51) wherewith the lid (29) is provided, with a corresponding cavity arranged in an elastically deformable element (53) joined to the said pan-shaped element (28) of the said device (10).

11. Apparatus according to one of claims 6 to 10, characterized in that the said device (10) capable of providing the values of the pressure of the liquid includes resilient means (23) which transmit to the said movable element (22) a thrust exerted towards the said reference element (21) in a radial direction relative to the said section (8) of the tube (6) and in an axial direction relative to the said piston (33).

12. Apparatus according to claim 11, characterized in that there is associated with the said piston (33) a lower cylindrical element (36) whose relative axial position is noted by the measurement means (24).

13. Apparatus according to claim 12, characterized in that the said resilient means (23) include a coil spring (46) coaxial with the said lower cylindrical element (36).

14. Apparatus according to claim 12 or 13, characterized in that the said lower cylindrical element (36) is provided at one end with a permanent magnet and that the said measurement means (24) include at least one electromagnetic-type transducer.

15. Apparatus according to claim 14, characterized in that the said electromagnetic transducer is a Hall effect transducer.

16. Apparatus according to claim 14, characterized in that the said electromagnetic transducer is a switch with magnetic action contacts.

17. Apparatus according to any one of claims 1 to 13, characterized in that the said measurement means (24) include a differential transformer whose connection depends on the position of the said movable element (22).

18. Apparatus according to any one of claims 1 to 13, characterized in that the said measurement means (24) include a potentiometer-type transducer.

19. Apparatus according to any one of claims 1 to 13, characterized in that the said measurement means (24) include at least an optoelectronic-type transducer.

## Patentansprüche

1. Apparat (1) für die extrakorporale Zirkulation von Blut mit einer Rohrleitung (2), einer Pumpe (3) und einer Einrichtung zum Messen des Blutdrucks in der Rohrleitung (2), wobei die Einrichtung folgendes aufweist:
- ein Bezugselement (21) und ein bewegliches Element (22), zwischen denen ein Teil der Rohrleitung (2) angeordnet werden kann, wobei dieser Teil stromauf der Pumpe (3) angeordnet ist,
- eine Meßeinrichtung (24), die die gegenseitige Stellung des beweglichen Elements (23) gegenüber dem Bezugselement (21) ermittelt, und die ein elektrisches Signal in Abhängigkeit von der verformung des angegebenen Teils abgibt, die in Betrieb durch den Druck des Bluts verursacht wird,
dadurch **gekennzeichnet,**
daß die Pumpe (3) eine Schlauchpumpe mit einem Rotor (4) ist, der mittels Rollen (5) auf die Außenfläche eines elastisch verformbaren Rohrs (6) wirkt, das mit der Rohrleitung (2) in Reihe geschaltet ist, und daß der Teil der Rohrleitung (2), an dem die Druckmeßeinrichtung montiert ist, durch einen stromauf von der Pumpe (3) gelegenen Abschnitt (8) des Rohrs (6) gebildet ist, der der Wirkung der Rollen (5) nicht ausgesetzt ist.

2. Apparat nach Anspruch 1,
dadurch gekennzeichnet,
daß die Vorrichtung (10) eine Einrichtung (15) aufweist zum Vergleichen des elektrischen Signals mit einem elektrischen Bezugssignal und zum Abgeben eines Alarmsignals entsprechend der Ermittlung eines gegebenen Unterschieds des elektrischen Signals gegenüber dem Bezugssignal.

3. Apparat nach Anspruch 2,
dadurch gekennzeichnet,
daß die Einrichtung zum Vergleichen und Abgeben eines Alarmsignals eine Vergleichsschaltung (15) mit Schwellenwert aufweist.

4. Apparat nach einem der Ansprüche 2 oder 3,
gekennzeichnet
durch eine Einrichtung (16) zum Steuern des Bezugssignals.

5. Apparat nach Anspruch 4,
dadurch gekennzeichnet,
daß die Steuereinrichtung ein Potentiometer (16) und einen mit der Vergleichsschaltung (15) verbundenen Schieber aufweist.

6. Apparat nach einem der vorhergehenden Ansprüche,
dadurch gekennzeichnet,
daß das Bezugselement (21) im wesentlichen durch einen Deckel (29) eines schalenförmigen Körpers (28) gebildet ist, in dem der Abschnitt (8) des Rohrs (6) aufgenommen ist, wobei das bewegliche Element (22) im wesentlichen durch einen Kolben (33) gebildet ist, der in einem Loch (32) axial verschiebbar ist, das in einer Wand an der Basis des schalenförmigen Körpers und in einer dem Deckel (29) gegenüberliegenden Position ausgebildet ist.

7. Apparat nach Anspruch 6,
dadurch gekennzeichnet,
daß der Kolben (33) einen Kopf (34) mit einer zentralen kreisförmigen ebenen Fläche aufweist, die mit einer entsprechenden Außenfläche des Abschnitts (8) des Rohrs (6) zusammenarbeiten kann.

8. Apparat nach Anspruch 7,
dadurch gekennzeichnet,
daß der Deckel (29) in dem nach innen gewandten Teil eine im wesentlichen zylindrische Erhöhung aufweist, die eine kreisförmige ebene Fläche bildet ähnlich derjenigen, die durch den vergrößerten Kopf (34) des Kolbens (33) gebildet ist.

9. Apparat nach einem der Ansprüche 6 bis 8,
dadurch gekennzeichnet,
daß die Verbindung des Deckels (29) mit dem schalenförmigen Element (28) durch Einrasten erfolgt.

10. Apparat nach Anspruch 9,
dadurch gekennzeichnet,
daß die Rastverbindung erzielt wird durch Verwendung eines Vorsprungs (51) des Deckels (29) zusammen mit einer entsprechenden Vertiefung (52) in einem elastisch verformbaren Element (53), das mit dem schalenförmigen Element (28) der Vorrichtung (10) verbunden ist.

11. Apparat nach einem der Ansprüche 6 bis 10,
dadurch gekennzeichnet,
daß die Vorrichtung (10) für die Lieferung der Druckwerte der Flüssigkeit eine elastische Einrichtung (23) aufweist, die auf das bewegliche Element (22) einen Schub überträgt, der auf das Bezugselement (21) in radialer Richtung gegenüber dem Abschnitt (8) des Rohrs (6) und in axialer Richtung gegenüber dem Kolben (33) wirkt.

12. Apparat nach Anspruch 11,
dadurch gekennzeichnet,
daß der Kolben (33) mit einem zylindrischen unteren Element (36) verbunden ist, dessen axiale relative Position von der Meßeinrichtung (24) ermittelt wird.

13. Apparat nach Anspruch 12,
dadurch gekennzeichnet,
daß die elastische Einrichtung (23) eine zum zylindrischen unteren Element (36) koaxiale Schraubenfeder (46) aufweist.

14. Apparat nach Anspruch 12 oder 13,
dadurch gekennzeichnet,
daß das zylindrische untere Element (36) an einem Ende mit einem Permanentmagnet versehen ist, und daß die Meßeinrichtung (24) wenigstens einen elektromagnetischen Wandler aufweist.

15. Apparat nach Anspruch 14,
dadurch gekennzeichnet,
daß der elektromagnetische Wandler ein Wandler mit Hall-Effekt ist.

16. Apparat nach Anspruch 14,
dadurch gekennzeichnet,
daß der elektromagnetische Wandler ein Schalter mit magnetisch wirkenden Kontakten ist.

17. Apparat nach einem der Ansprüche 1 bis 13,
dadurch gekennzeichnet,
daß die Meßeinrichtung (24) einen Differentialübertrager aufweist, dessen Kopplung von der Position des beweglichen Elements (22) abhängt.

18. Apparat nach einem der Ansprüche 1 bis 13,
dadurch gekennzeichnet,
daß die Meßeinrichtung (24) einen Wandler der Potentiometerbauart aufweist.

19. Apparat nach einem der Ansprüche 1 bis 13,
dadurch gekennzeichnet,
daß die Meßeinrichtung (24) wenigstens einen Wandler der elektronenoptischen Bauart aufweist.
